(19) Europäisches Patentamt

European Patent Office

Office européen des brevets

(11) Publication number: **0 208 521 B1**

# EUROPEAN PATENT SPECIFICATION

(45) Date of publication of patent specification: **13.11.91**

(51) Int. Cl.⁵: **G06F 15/20**

(21) Application number: **86305198.3**

(22) Date of filing: **04.07.86**

(54) **Improved automated mean arterial blood pressure monitor with data enhancement.**

(30) Priority: **05.07.85 US 751826**

(43) Date of publication of application:
**14.01.87 Bulletin 87/03**

(45) Publication of the grant of the patent:
**13.11.91 Bulletin 91/46**

(84) Designated Contracting States:
**AT BE CH DE FR GB IT LI LU NL SE**

(56) References cited:
**WO-A-85/00099**
**FR-A- 2 498 071**
**GB-A- 2 092 309**
**US-A- 4 190 886**
**US-A- 4 407 297**

(73) Proprietor: **CRITIKON, INC.**
**4110 George Road**
**Tampa Florida 33607(US)**

(72) Inventor: **Ramsey, Maynard III**
**903 Golfview**
**Tampa, FL 33609(US)**
Inventor: **Hood, Rush W., Jr.**
**15615 Gardenside Lane**
**Tampa, FL 33624(US)**
Inventor: **Medero, Richard**
**2410 Blindpond Avenue**
**Lutz, FL 33549(US)**

(74) Representative: **Jones, Alan John et al**
**CARPMAELS & RANSFORD 43 Bloomsbury**
**Square**
**London, WC1A 2RA(GB)**

## Description

This invention relates to automated blood pressure measuring apparatus and, more particularly to stored program controlled monitors employing the oscillometric method of detection characterised by data purification and enhanced systolic, diastolic and mean blood pressure determination.

Reference is hereby made to the following concurrently filed co-pending European patent applications:
IMPROVED SPHYMOMANOMETRIC CUFF PRESSURISING SYSTEM, EP-A-0207805;
OSCILLOMETRIC BLOOD PRESSURE MONITOR EMPLOYING NON-UNIFORM PRESSURE DECREMENTING STEPS, EP-A-0208520;
IMPROVED AUTOMATED SYSTOLIC BLOOD PRESSURE MONITOR WITH DATA ENHANCEMENT, EP-A-0207806;
IMPROVED AUTOMATED DIASTOLIC BLOOD PRESSURE MONITOR WITH DATA ENHANCEMENT, EP-A-0207807.

Automated blood pressure monitoring has rapidly become an accepted and, in many cases, essential aspect of human and veterinary treatment. Such monitors are now a conventional part of the patient environment in emergency rooms, intensive and critical care units, and in the operating theatre.

The so-called oscillometric method of measuring blood pressure is one of the most popular methods in commercially available systems. This method relies on measuring changes in arterial counterpressure, such as imposed by an inflatable cuff, which is controllably relaxed or inflated. In some cases the cuff pressure change is continuous, and in others it is incremental. In substantially all, a transducer monitors arterial counterpressure oscillations, and processing apparatus converts select parameters of these oscillations into blood pressure data.

The principles of the present invention are described in US-4360029 and US-4349034, which are commonly assigned with the instant invention. Both patents disclose apparatus and methods for artifact rejection in oscillometric systems. In accordance with the principles described in these patents, an inflatable cuff is suitably located on the limb of a patient, and is pumped up to a predetermined pressure. Thereupon, the cuff pressure is reduced in predetermined fixed decrements, at each level of which pressure fluctuations are monitored. These typically consist of a DC voltage with a small superimposed variational component caused by arterial blood pressure pulsations (referred to herein as "oscillatory complexes"). Therefore, after suitable filtering to reject the DC component and to provide amplification, pulse peak amplitudes above a given threshold are measured and stored. As the decrementing continues, the peak amplitudes will normally increase from a lower amount to a relative maximum, and thereafter will decrease. The lowest cuff pressure at which the oscillations have a maximum peak value is representative of mean arterial pressure. The cuff pressures obtained when stored oscillation complex pulse peak amplitudes bear predetermined fractional relationships with the largest stored peak corresponding to the subject's systolic and diastolic pressures.

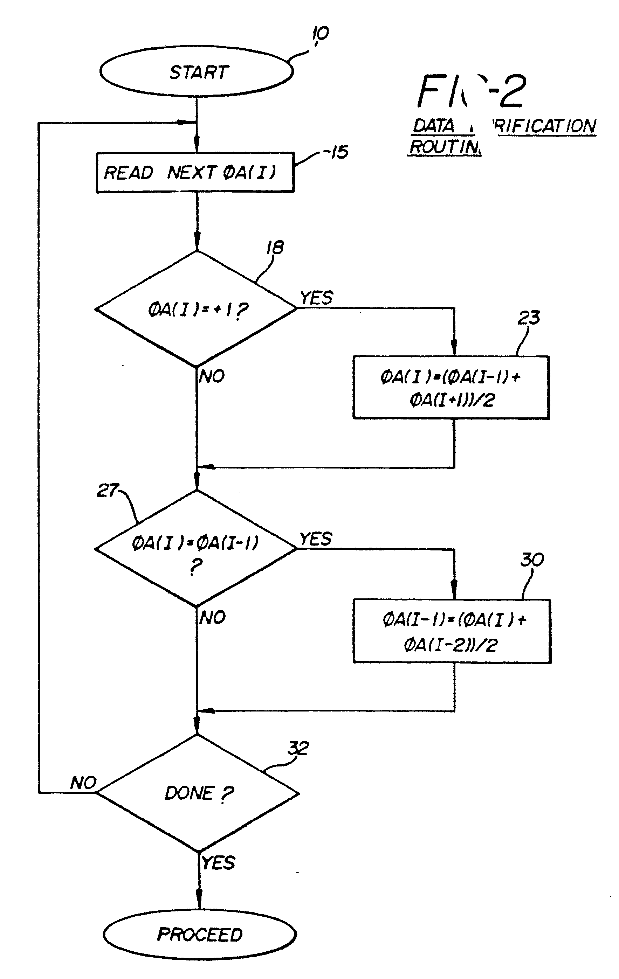

US-4360029 and US-4349034 describe the rejection of artifact data to derive accurate blood pressure data. Indeed, as is apparent from Fig. 2 of both patents, the most substantial portion of the measurement cycle (denominated "T3") is devoted to the execution of complex detection at the various pressure levels, measurement of signal peaks of true complexes, and processing those peaks in accordance with artifact rejection algorithms. Notwithstanding such efforts, the signal peak data collected sometimes incorporates data errors, i.e., a data pattern inconsistent with the above described typical physiological response pattern of a subject as the artery occluding cuff pressure monotonically decreases.

In EP-A-0208520 which has the same priority date as the present invention, oscillometric blood pressure measurements are effected with non-uniform, cuff pressure-dependent pressure decrements between successive oscillatory complex peak measuring intervals. Such a method of effecting oscillometric blood pressure measurements is facilitated by systolic, diastolic and mean blood pressure determining algorithms not heretofore employed.

It is an object of the present invention to provide improved oscillometric blood pressure determining apparatus and methodology.

More specifically, it is an object of the present invention to purify the oscillatory complex peak amplitude data ensemble employed for blood pressure determination.

Yet another object of the present invention is the provision of improved algorithms, methodology and apparatus for determining systolic, diastolic and mean arterial blood pressure.

A blood pressure cuff is applied about a subject's artery, and inflated above the systolic level thus fully occluding the artery for a full heart cycle. The cuff pressure is thereafter reduced to permit an increasing flow through the progressively less occluded artery, and a measure of the peak amplitudes of the successively encountered oscillatory complexes stored in memory. Also retained is the cuff pressure obtaining for each stored complex peak.

In accordance with varying aspects of the present invention, the stored complex peak-representing data set is corrected for aberrations; and improved data processing operates on the stored (and advantageously corrected) pulse peak data and the corresponding cuff pressure information to determine the subject's systolic, diastolic and mean arterial pressure.

According to the invention, there is provided an automated blood pressure monitor comprising an inflatable cuff; means for inflating and deflating said cuff; pressure transducer means coupled to said cuff for cuff pressure measurement; means responsive to said cuff pressure measurement for generating a signal representing blood pressure pulses; complex peak storing means for storing values characterising the peak amplitudes of said detected pulses at different cuff pressures; cuff pressure storing means for storing the cuff pressures associated with said cuff pressure pulse peak signals; and mean arterial pressure determining means, characterised in that: said mean arterial pressure determining means comprises: means for locating the maximum cuff pressure complex peak amplitude stored in said complex peak storing means; means for locating a successor peak value stored in said complex peak storing means next occurring after said maximum complex peak amplitude; means for selecting from said complex peak storing means, plural peak amplitudes generated at higher cuff pressures than that associated with said maximum complex peak amplitude; and determining means for determining mean arterial pressure from: said plural peak amplitudes; and cuff pressures stored in said cuff pressure storing means associated with: said selected plural peak amplitudes; and said successor peak value.

According to a further aspect of the present invention, there is provided means for identifying and maintaining the associated cuff pressure decrement levels; means for interpolating the peak amplitudes from the identified associated cuff pressure decrement levels; and said determining means further comprises: means for developing mean arterial pressure as a predetermined weighted average of the cuff pressure corresponding to the interpolated peak amplitude and at least one of the identified and maintained cuff pressure decrement levels.

The preferred embodiment of the invention will now be described with reference to the accompanying drawings, in which:

Fig. 1 is a timing diagram illustrating data generation and correction during an illustrative measurement cycle for oscillometric blood pressure determination in accordance with the principles of the present invention;

Fig. 2 is a flow chart illustrating data purification for improved oscillometric blood pressure determination;

Fig. 3 depicts oscillation amplitude processing for a systolic blood pressure measurement in accordance with the present invention;

Fig. 4 is a program flow chart for the systolic blood pressure measurement typified in Fig. 3;

Fig. 5 illustrates blood pressure interpolation for the processing mode of Figs. 3 and 4 (and by analogy for Figs. 6-9 as well);

Fig. 6 depicts oscillatory complex measuring wave forms illustrating diastolic blood pressure determination in accordance with the present invention;

Fig. 7 is a program flow chart illustrating the diastolic blood pressure measurement typified by Fig. 6;

Fig. 8 is a timing diagram depicting oscillatory complex peak amplitude processing for mean arterial pressure measurements in accordance with the present invention; and

Fig. 9 is a program flow chart illustrating the mean arterial pressure determination typified by Fig. 8.

US-4360029, US-4349034, US-4543962, and EP-A-0208520 describe in detail the basic oscillometric method of measuring blood pressure forming a background and a starting point for the instant invention.

An artery-occluding cuff is disposed on the subject, e.g., about a subject's upper arm over the brachial artery. At the inception of a measuring cycle, the cuff is inflated to a pressure which fully occludes the brachial artery, i.e., prevents blood from flowing therethrough at any point in the heart cycle. The cuff is then progressively deflated, as in discrete steps. A pressure transducer is coupled to the internal cuff pressure and provides an analog signal characterising the blood pressure oscillatory complexes when they begin to occur (i.e., when the maximum heart pressure corresponding to contraction of the heart's left ventricle exceeds the instantaneously obtaining artery-occluding cuff pressure). The peak values of the complex signals are determined in hardware or software.

As the measurement cycle progresses, the peak amplitude of the blood pressure complexes generally become monotonically larger to a maximum and then become monotonically smaller as the cuff pressure continues toward deflation. The peak amplitude of the cuff pressure oscillation complexes, and the corresponding occluding-cuff pressure values are retained in computer memory. US-4360029, US-4349034, US-4543962 and EP-A-0208520 illustrate previously employed algorithms for processing the stored blood pressure complex peak values and concomitant pressure values to yield the subject's mean arterial pressure. These patents and applications also furnish detailed procedures for measuring oscillatory complex peaks; procedures for testing complexes and rejecting bad data associated with measurement-impeding artifacts (such as motion) during a measurement cycle, and the like.

The oscillometric blood pressure measurements as typified by the disclosed principles are effected under stored program control, as via a microprocessor operative in conjunction with a program containing read only memory (ROM or PROM), and a variable content random access memory (RAM) which stores the cuff pressures, oscillatory complex peak amplitudes, and other processing operand variables. The microprocessor receives the cuff pressure readings generated by the pressure transducer, for example as processed by a peak detector, amplifier and analog-to-digital convertor, and supplies all output control signals required, e.g., to open and close one or more cuff deflating valves.

The cuff may be inflated directly by an air pump; and deflated in fixed, discrete steps under microprocessor control. Alternatively, the cuff may be principally or entirely inflated by the pressurised contents of an air reservoir; and/or deflation may proceed in variable, cuff pressure-dependent steps via selected one or ones of plural deflating valves. These latter alternatives achieve the desideratum of condensing the time required for a composite measurement cycle of operation.

Also, there are alternative procedures for measuring the oscillatory complex peak amplitude at any prevailing cuff pressure. In one mode heretofore employed, plural (e.g., two) complex peaks are measured at each cuff pressure step during cuff deflation, and their average used as the peak value. Since the peaks should be approximately equal, any marked disparity (e.g., > 20%) signals that some artifact error occurred and the data is rejected. In a fast ("stat") mode, after several intervals of qualifying (close or equal peak values) companion complexes are detected to develop measurement confidence, only one pulse is required during succeeding cuff deflation intervals thus speeding the composite measurement period.

As alluded to above, it is sometimes the case when blood pressure complexes are being examined for peak amplitude at any occluding pressure level that improper data is developed. There are varying causes for such aberrations. Perhaps the most common is spurious motion by the subject which generates an inadvertent pressure impulse in the cuff which is sensed by the pressure transducer which may be then incorrectly reflected in the blood pressure measurement. Other causes include varying sources of interfering electrical noise or internal cardiac or respiratory changes in the subject. When a false complex peak amplitude value is generated, it is discarded by the composite measuring apparatus and a discard-signalling value (e.g., + 1) retained in its place in memory.

A second form of spurious data occurs when the pattern of stored pulse peak values departs from the physiologically mandated sequence of values which progressively increase to a peak and then progressively decrease.

Attention will now be directed to data processing under stored program control for purifying the data collected by the above-described blood pressure measuring apparatus. Further, specific illustrative efficient algorithms are discussed for in fact determining the subject's systolic, diastolic and mean arterial blood pressures. Such data processing may be effected on any computing equipment, preferably digital microprocessors such as commercially available from a number of vendors. The program instructions and sequences presented below are for illustrative purposes only. Such instructions may in fact be implemented in any of diverse program languages and sequences readily apparent to those skilled in the art. In the signal processing below discussed, processing variables have the following significance:

## I.  Variables Employed For All Data Processing Below Discussed

### Variable

### Functional Quantity Represented

CP(I)

The cuff pressure, measured by the transducer pneumatically coupled to the artery occluding cuff, obtaining during the i-th deflation step. CP(I) is an indexed array, i.e., there exists a plurality of values for CP(I) characterizing each of the $\underline{i}$ deflation steps.

$\Phi A(I)$

The peak amplitude of the oscillometric oscillation (i.e., the complex peak amplitude) occurring at the i-th step. Where multiple complexes are measured during each prevailing deflation pressure, $\Phi A(I)$ is the average of two (or more) peak amplitudes during the i-th step. $\Phi A(I)$ is an indexed array.

$\Phi A(MAX)$

The peak value of the array of averaged oscillatory blood pressure complex amplitudes.

MAX

The time interval when the peak complex $\Phi A(MAX)$ occurred.

## II. Variables Specific To Systolic Pressure Measurement

| Variable | Functional Quantity Represented |
|---|---|
| LVL | An intermediate processing variable representing a predetermined fraction of $\Phi A(MAX)$. |
| SYS | The subject's measured systolic pressure. |

## III. Diastolic Pressure Variables

| Variable | Functional Quantity Represented |
|---|---|
| UDLVL and LDLVL | Intermediate processing variables each representing a different fraction of $\Phi A(MAX)$. |
| DIAU, DIAL | Intermediate processing variables representing upper and lower interpolated diastolic pressure computational variables. |
| DIA | The subject's measured diastolic pressure. |

## IV. Mean Arterial Pressure Processing Variables

| Variable | Functional Quantity Represented |
|---|---|
| AMP | The complex pulse peak for the deflation interval following that |

6

for which the pressure oscillation amplitude was the maximum.

MAPL     An intermediate processing variable employed in the final mean arterial pressure computation.

MAP     The subject's mean arterial blood pressure.

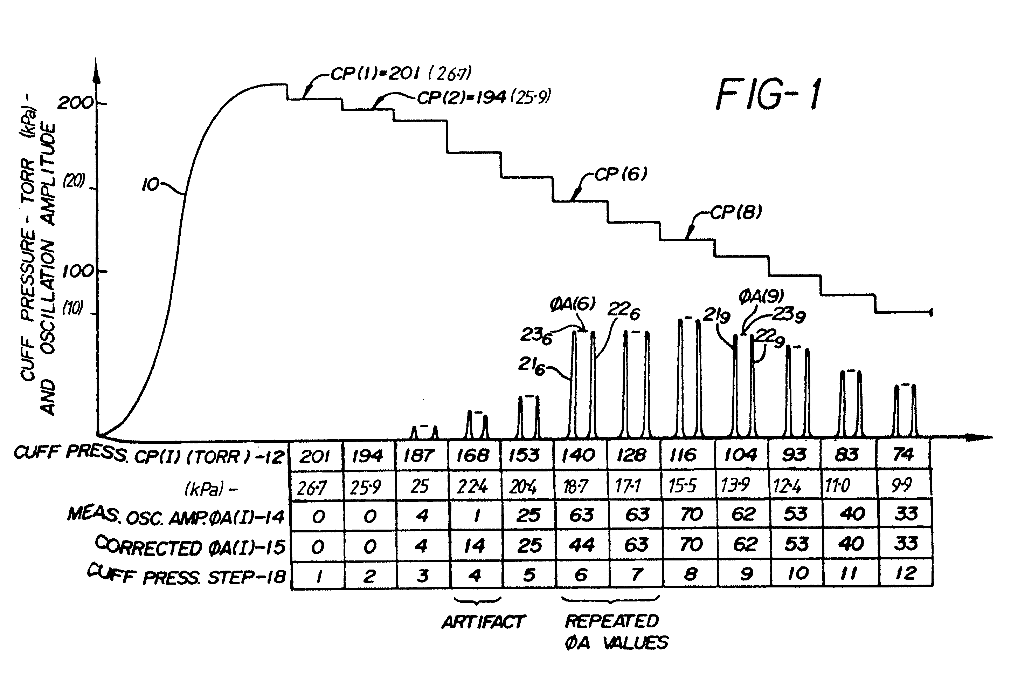

Turning now to Fig. 1, there is depicted wave forms with associated data characterizing the generation of data for an oscillatory blood pressure measurement - and purging (overcoming) bad data constituents. In accordance with the above discussion, the cuff artery occluding pressure for a measurement cycle, as measured by the cuff-associated transducer is characterized by a wave form 10. The cuff pressure rapidly increases to a maximum above the subject's systolic pressure, and is then deflated in a sequence of steps to a point below the diastolic pressure. The sequence of cuff deflation steps is indicated by the time interval signalling digits 1,2, ... , (lowest row 18 in the data table portion of Fig. 1). The internal pressure characterizing the cuff pressure at each step i is given by the data array CP(1),CP(2), ... (upper data table row 12).

Each step (time interval) is made sufficiently long to include at least two heart beats. Accordingly, at least two cuff pressure complex pulses $21_i$ and $22_1$ are measured during each interval after such pulses begin. Legends have been applied to pulses occurring during deflation steps 6 and 9 to avoid clutter and loss of clarity in Fig. 1. No pulses are measured during the first and second pressure steps (time intervals), it being assumed that the cuff pressure [CP(1) = 26.7 kPa (201 Torr), and CP(2) = 25.9 kPa (194 Torr)] are sufficient during these periods to obviate blood flow through the subject's artery for the full heart cycle. During the following intervals 3,4 ... , two oscillometric complex pulses 21 and 22 are generated and measured, the two pulses having an average peak amplitude 23 (the processor variable array value initially stored in $\phi A(I)$). The measured oscillation amplitude array ($\phi A(I)$) is shown in row 14 of the Fig. 1 data table for each time interval.

As above noted, assuming a perfect measurement, the oscillation pressure amplitude $\phi A(I)$ data row would not contain any +1 values which signify an impeded measurement. Further, the data pattern in the second row of the data table for the oscillation amplitudes would exhibit a pattern of successively increasing numbers to a peak value, followed by progressively decreasing values - all without adjacent equal $\phi A(I)$ values. To the extent that any $\phi A(I) = 1$ values are stored, or to the extent that the progressively increasing/decreasing pattern does not obtain, the data processing in accordance with the instant invention functions to compute appropriate corrected $\phi A(I)$ values (the third data table row 15 in Fig. 1) for the oscillation amplitude entries requiring correction.

In overview, where any $\phi A(I) = 1$ values exist, they are replaced by the average value of the oscillation amplitude in the two contiguous storage cells, i.e.,

$$\phi A(I) = (\phi A(I-1) + \phi A(I+1))/2. \qquad \text{Eq. 1.}$$

Correspondingly, where two contiguous oscillation amplitudes have the proscribed equal values, the first of the contiguous equal pair is replaced by the average of the amplitudes of the complex peaks measured at the next lower and next higher occluding cuff pressures. See, for example, Eq. 1 and, more particularly, the comparable relationship in functional block 30 of Fig. 2.

Data flow effecting the data purification algorithm above-discussed is set forth in the program flow chart of Fig. 2. Fig. 2 operates on the measured average oscillation amplitudes (the second data table row 14 in Fig. 1) and generates the corrected $\phi A(I)$ values shown in the third row 15 of Fig. 1. To this end, proceeding from a start block 10 (Fig. 2), step 15 reads the next value $\phi A(I)$ (proceeding toward the right along the Fig. 1 data table row 14) and test 18 determines whether the value stored in $\phi A(I)$ equals the error-signalling value +1. If as is the usual case it does not (indicating that the value measured was presumptively free of artifacts and the like), control passes to equality test 27. However, if the contents of $\phi A(I)$ did equal +1 ("YES" branch of test 18), functional block 23 implements Eq. 1, i.e., replaces the + 1 former contents of memory cell φA(I) corresponding to cuff pressure CP(I) with the average value of the oscillation amplitude measured at the next lower (φA(I-1)) and next higher non-plus one (φA(I+1)) deflation steps. The processing steps 18 and 23 thus purge the measured pressure peak amplitude storage contents (row 14 of the Fig. 1 data table) of all + 1 values, replacing these by the average value of the measurements made during immediately adjacent deflation steps (corrected φA(I) contents being illustrated in row 15).

Test 27 next examines the current operand φA(I) for the proscribed equality with the previous value φA-(I-1). If, as is normally the case, the contents of φA(I) and φA(I-1) differ ("NO" branch from test 27), processing flows to test 32 to determine whether each of the N elements of φA(I) have been processed. If they have not, control returns to block 15 to read in and process the next φA(I) element of the array in the third row 15 of the Fig. 1 data table. When all elements have been processed, control exits from the Fig. 2 data purification routine to data processing point 33 to proceed with the next (unrelated) task for the microprocessor.

If a data error has occurred ("YES" output of test 27 signalling that a data value φA(I) equaled the previous value), control passes to step 30 which replaces the assumed erroneous element φA(I-1) - (the value which should differ from φA(I) but did not) with the average of the two immediately contiguous elements, as by

$$\phi A(I-1) = (\phi A(I) + \phi A(I-2))/2. \qquad \text{Eq. 2.}$$

Accordingly, the data purification routine depicted in Fig. 2 and above-discussed replaces all error reading signifying φA(I) = 1 values with an interpolated estimated value; and purges the data table row 14 φA(I) array of data of any contiguous equal values. The corrected set of φA(I) is shown in row 15 of the Fig. 1 data table. Thus, for example, the oscillation amplitude value during the cuff pressure step (time interval) "4" is corrected from the error-signalling + 1 value to a peak amplitude 14, representing the average of measurements 4 and 25 at cuff pressures 25 kPa (187 Torr) and 20.4 kPa (153 Torr) during the immediately contiguous time intervals 3 and 5. Similarly, the first (pressure step 6) of two equal measured oscillation amplitude pulses of value 63 during periods 6 and 7, corresponding to occluding cuff pressures of 18.7 kPa (140 Torr) and 17.1 kPa (128 Torr), is corrected to a value of 44 representing the average of the contiguous measured amplitudes of 63 and 25 units.

The corrected array φA(I) as represented by row 15 in Fig. 1 thus comprises values from which each of the systolic, diastolic and mean arterial blood pressures may be determined either in accordance with the improved algorithms below discussed or employing the algorithms of the above referenced patents and patent applications. The data purification above discussed provides more accurate measurements than was heretofore the case; and also permits blood pressures to be determined more quickly, obviating the need for repeated deflation steps when unacceptable artifact or noise corrupted data is sensed.

Attention will now be shifted to the particular method pursuant to which the stored cuff pressure CP(I) and corrected blood pressure peak value φA(I) information in the first row and in row 15 of Fig. 1 is employed in accordance with other aspects of the present invention to measure a subject's systolic, diastolic and mean arterial blood pressures.

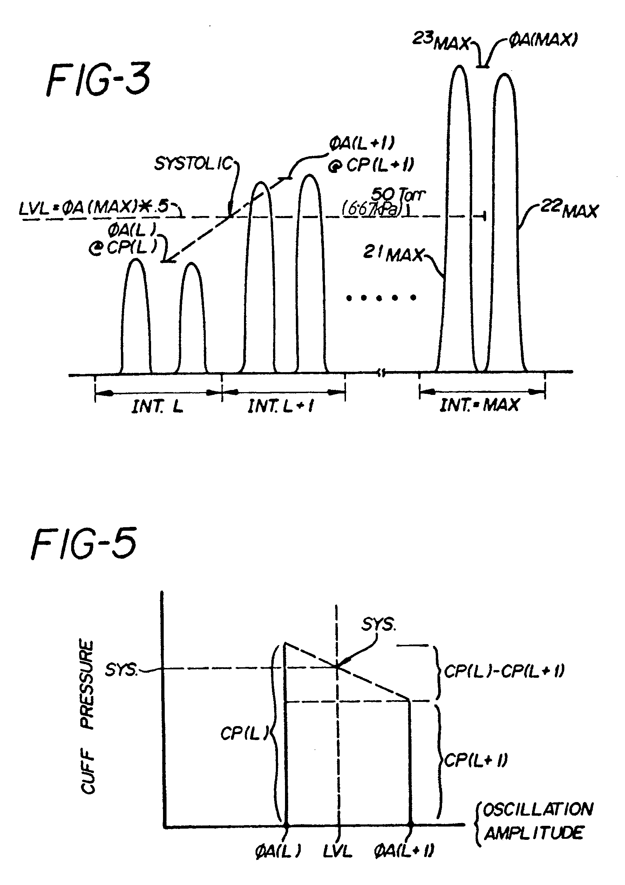

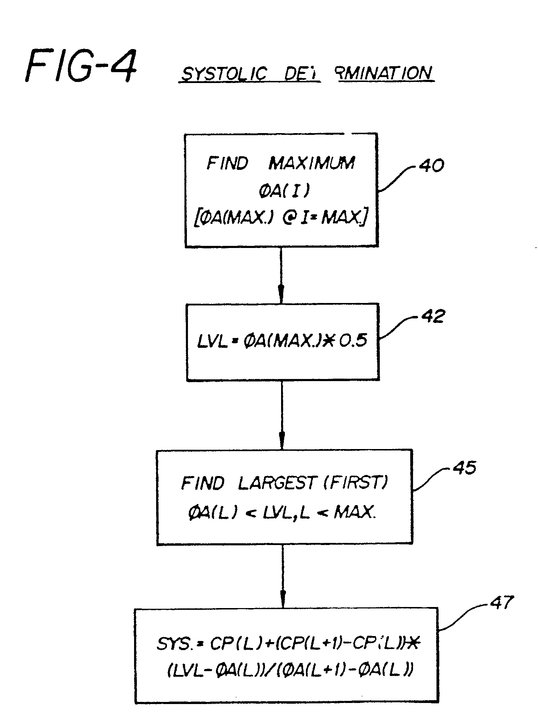

Pulse complex wave form processing typifying systolic blood pressure determination is illustrated in Fig. 3, and a flow chart for the underlying data processing is set forth in Fig. 4. In overview, systolic pressure is determined by:

(a) Finding the amplitude (φA(MAX)) of the largest blood pressure oscillatory complex (which occurs at the time interval MAX);

(b) Finding an amplitude level (LVL) equal to a predetermined fraction of the peak value φA(MAX). We have found a value of 0.5 to be satisfactory for normal processing with something less (e.g., 0.45) for stat (rapid deflation and/or single pulse) operation;

(c) Examining the corrected oscillation amplitude (φA(I)) values (third row 15 in the Fig. 1 data table) starting at the MAX interval and proceeding toward the higher cuff pressure direction (i.e., to the left in Figs. 1 and 3) to find the two contiguous oscillation amplitudes for which

$$\phi A(L) < MAX^*0.5 < \phi A(L+1); \qquad \text{Eq. 3.}$$

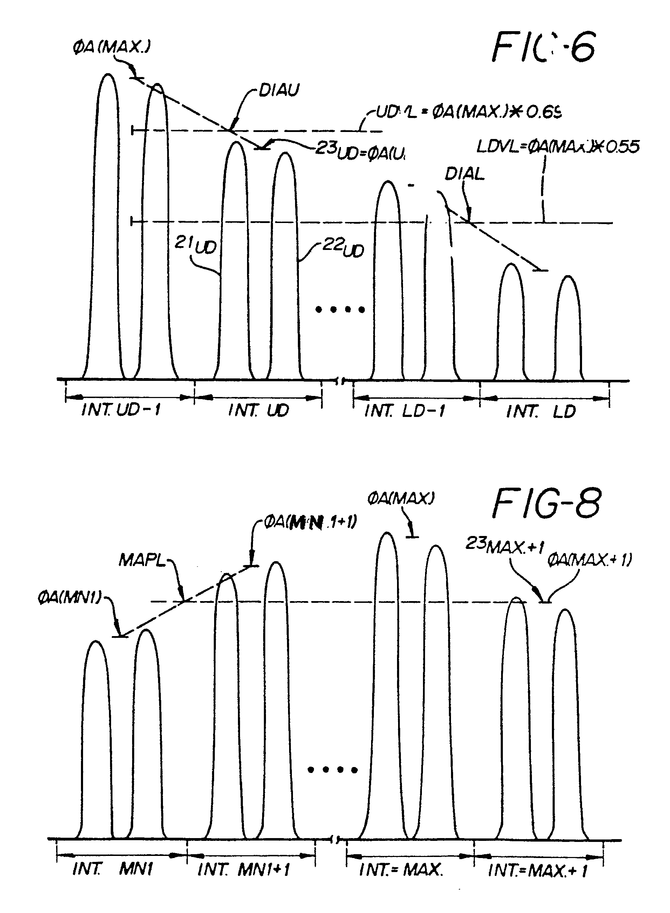

(d) Computing the interpolated cuff pressure (between CP(L) and CP(L+1)) assuming a linear variance in oscillation amplitude and cuff pressure between the intervals L and L+1. This per se well known linear trapezoidal interpolation is graphically depicted in Fig. 5. The interpolated cuff pressure directly corresponds to the subject's systolic blood pressure (SYS). Expanding upon the systolic pressure determining methodology set forth above, the cuff pressure interval I = MAX when the largest oscillation amplitude peak occurs is determined in any per se well known manner, (step 40 of the Fig. 4 flow chart corresponding to the interval MAX in Fig. 3).

Thus, for example, the following schematic BASIC sequence will suffice as illustrative to find the interval MAX:

$$\phi AMAX = \phi A(1) \quad \text{Eq. 4.}$$

$$MAX = 1 \quad \text{Eq. 5.}$$

$$F)R \; K = 2 \; TO \; N \quad \text{Eq. 6.}$$

$$IF \; \phi A(K) < \phi AMAX \; GOTO \; 70 \quad \text{Eq. 7.}$$

$$\phi AMAX = \phi A(K) \quad \text{Eq. 8.}$$

$$MAX = K \quad \text{Eq. 9.}$$

$$70 \; NEXT \; K \quad \text{Eq. 10.}$$

In brief, Equations 4 and 5 make an initial assumption that the peak value occurred during the first interval and load a provisional peak value storing variable $\phi AMAX$ with the value $\phi A(1)$. For an assumed N-time interval measurement, the loop between Equations 6 and 10 sequentially examines every element of the $\phi A(I)$ array from 2 to N, updating $\phi AMAX$ only when the value $\phi A(K)$ - (K being the loop index) exceeds the previously assumed $\phi AMAX$ value. When the processing exits from the loop following instruction 70 in Equation 10 the variable MAX contains the value of I such that $\phi A(MAX)$ is the largest value in the array.

The next following step 42 sets a variable LVL equal to the predetermined fraction of the peak amplitude $\phi A(MAX)$ as by

$$LVL = \phi A(MAX)^*0.5. \quad \text{Eq. 11.}$$

The value LVL is shown by the dashed line 50 in Fig. 3.

The next following operation 45 finds the first time interval (L) preceding MAX for which the oscillation amplitude peak is less than LVL, i.e., less than one-half of the peak value $\phi A(MAX)$, thereby finding the two contiguous values (L, L + 1) having peak amplitudes which bound the value in LVL. Algorithms for conducting such a search are well known to those skilled in the art, e.g.,

$$FOR \; J = 1 \; TO \; MAX \quad \text{Eq. 12.}$$

$$IF \; (\phi A(MAX-J)-LVL) < 0 \; GOTO \; 140 \quad \text{Eq. 13.}$$

$$NEXT \; J \quad \text{Eq. 14.}$$

$$140 \; L = MAX-J \quad \text{Eq. 15.}$$

Equations 12-15 simply comprise a DO or FOR-NEXT loop progressing from MAX-1 toward L = 1, exiting when the first sub-LVL value is obtained. The appropriate interval identification (MAX-J) is stored in the variable location L.

Finally, the value of the systolic pressure is estimated by assuming a linear variation in cuff pressure between the values CP(L) and CP(L + 1), and a linear variation between the corresponding oscillation amplitude $\phi A(L)$ and $\phi A(L + 1)$. Thus, in accordance with the per se well known trapezoidal interpolation equation, the systolic pressure SYS may be determined (step 47 of Fig. 4) by

$$SYS = CP(L) + \frac{(CP)(L+1-CP(L))*((LVL)-\Phi A(L))}{\Phi A(L+1)-\Phi A(A)} \qquad Eq. \ 16.$$

To illustrate employing the data of Fig. 1, 50% of the peak amplitude (9.33) is 4.67, and thus the pulse complex measurements of time intervals 5 and 6 are selected for systolic pressure computation. The Eq. 16 software interpolation implementation yields:

$$SYS = 20.4 + ((18.67\text{-}20.4)x(4.67\text{-}3.33)/(5.87\text{-}3.33)); \qquad Eq. \ 17.$$

$$= 19.5 \ kPa \qquad Eq. \ 18.$$

assuming three significant figures.

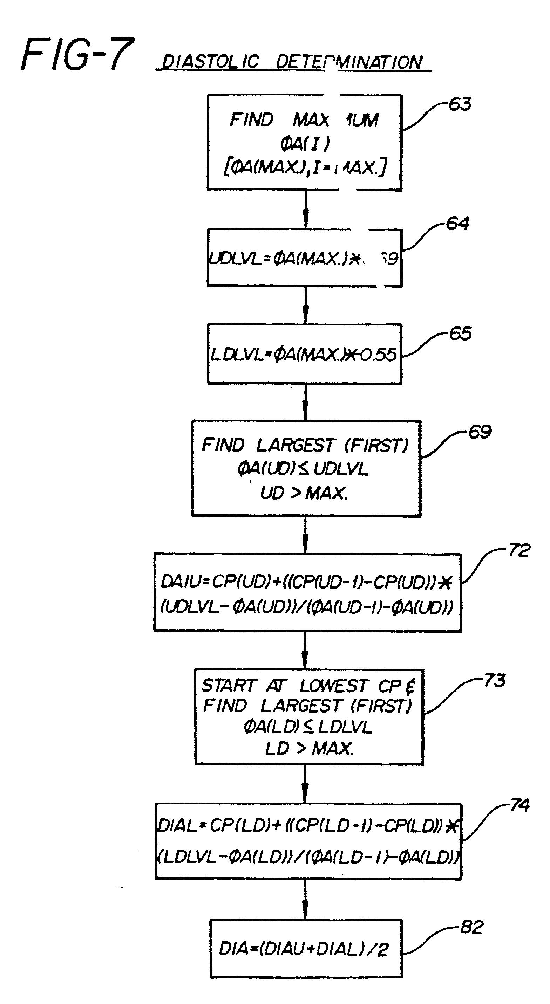

Pulse complex wave form processing characterizing diastolic blood pressure determination is illustrated in Fig. 6; and a flow chart for the underlying diastolic data processing algorithm is depicted in Fig. 7. In overview, diastolic pressure is determined by:

(a) the amplitude ($\phi A(MAX)$) of the complex (which occurs at the time interval MAX);

(b) Finding an amplitude level (UDLVL) equal to a first predetermined fraction of the peak value $\phi A$-(MAX). We have found a value of 0.69 to be satisfactory for normal processing and 0.72 for rapid ("stat") processing;

(c) Examining the corrected oscillation amplitude ($\phi A(I)$) buffer 15 (Fig. 1) starting at the MAX interval and proceeding toward the lower cuff pressure direction (i.e, to the right in Figs. 1 and 6) to find the two contiguous oscillation amplitudes for which

$$\phi A(UD) \leq MAX^*0.69 \leq \phi A(UD\text{-}1); \qquad Eq. \ 19.$$

(d) Finding the interpolated cuff pressure (between CP(UD-1) and CP(UD)) assuming a linear variation in oscillation amplitude and cuff pressure between the intervals UD-1 and UD (processing variable DIAU in Fig. 7);

(e) Examining the stored $\phi A(I)$ oscillation amplitude values at pressures starting at the lowest cuff pressure measured for a contiguous pair bounding the peat amplitude $\phi A(MAX)$ multiplied by a second factor lower than the first factor (e.g., 0.55), i.e., where

$$\phi A(LD) \leq MAX^*0.55 \leq \phi(LD\text{-}1); \qquad Eq. \ 20.$$

(f) Computing the interpolated cuff pressure between CP(LD) and CP(LD-1) corresponding to MAX times the 0.55 factor. This lower interpolated cuff pressure is associated with the variable designation DIAL; and

(g) Determining the subject's diastolic pressure (DIA) as the average of the upper and lower interpolated values DIAU and DIAL, i.e.,

$$DIA = (DIAU + DIAL)/2. \qquad Eq. \ 21.$$

The above-described procedure is illustrated in the blood pressure complex depiction of Fig. 6 and the Fig. 7 flow chart. The peak $\phi A(MAX)$ is first located as by the processing of Equations 4-10. The upper and lower peak amplitude fractions DIAU and DIAL are next determined (steps 64 and 65 of Fig. 7 corresponding to the labeled horizontal dash lines in Fig. 6). Step 69 then finds the first time interval (UD) following MAX at which the peak amplitude $\phi A(UD)$ is lower than the value stored in DIAU (as by processing analogous to that of Equations 12 through 15 replacing "MAX-J" with "MAX+J"). Thereafter, step 72 performs the trapezoidal interpolation analogous to that of Fig. 5, determining the cuff pressure (DIAU) corresponding to the UDLVL complex amplitude value. It is observed that the time interval UD-1 coincides with the interval MAX when the peak complex value occurred since, for the data case illustrated, the first pulse complex following MAX less than 0.69 x $\phi A(MAX)$ occurred in the next time interval MAX+1.

The functional steps 73 and 74 of Fig. 7 perform in a manner directly analogous to operations 69 and 72, locating the cuff pressure DIAL by interpolation for the intervals when the peak complex amplitudes bound the LDLVL value equal $\phi A(MAX)$ times 0.55. This latter search is conducted from $\phi A(i)$ at the lowest cuff pressure, then working toward higher cuff pressures. Finally, the subject's diastolic pressure (DIA) is computed as the average of the contents stored in DIAU and DIAL (step 82). To illustrate with a numerical example, again employing the data portion of Fig. 1,

$$DIAU = 11.1 + ((12.4\text{-}11.1)x(6.4\text{-}5.3))/(5.3\text{-}7.1) = 10.3 \text{ kPa} \qquad \text{Eq. 22.}$$

$$DIAL = 9.9 + ((11.1\text{-}9.9)x(5.1\text{-}4.4))/(4.4\text{-}5.3) = 9 \text{ kPa} \qquad \text{Eq. 23.}$$

$$DIA = (10.3 + 9)/2 = 9.65 \text{ kPa} \qquad \text{Eq. 24.}$$

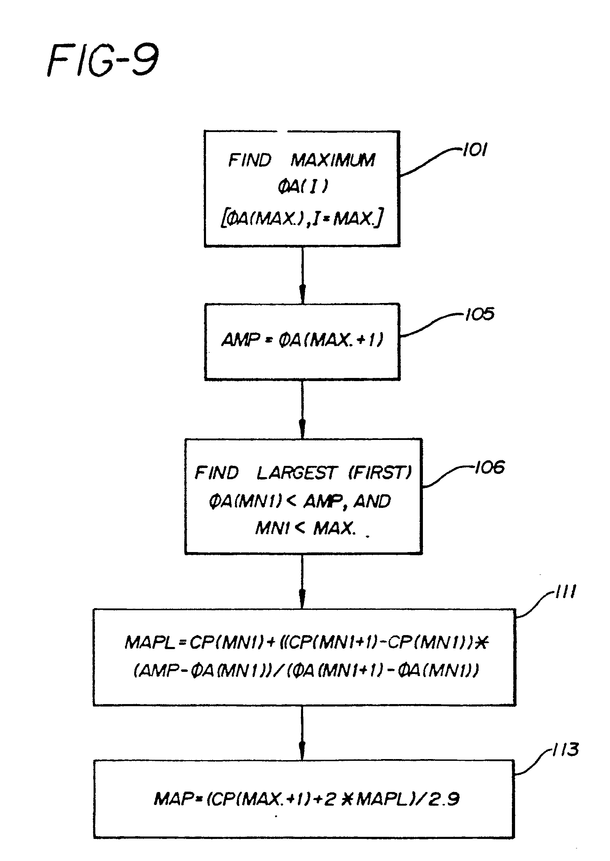

Finally, wave form processing illustrating mean arterial blood pressure measurement is shown in Fig. 8, and in flow chart form for the corresponding data processing in Fig. 9. In summary, mean arterial pressure is determined by:

(a) Finding the amplitude ($\phi A(MAX)$) of the largest blood complex (which occurs at the time interval MAX);

(b) Examining the cuff pressure values in the corrected register 15 (Fig. 1) for the interval MN1 yielding the first oscillation amplitude less than $\phi A(MAX + 1)$, i.e., the first cuff pressure to the left of the interval MAX which was less than the complex peak amplitude $\phi A(MAX + 1)$ occurring in the first interval following the time MAX. This satisfies the relationship

$$\phi A(MN1) \leq \phi A(MAX + 1) \leq \phi A(MN1 + 1); \qquad \text{Eq. 25.}$$

(c) An interpolation is then conducted between the intervals MN1 and MN1 + 1 for a cuff pressure MAPL corresponding to the oscillation amplitude value $\phi A(MAX + 1)$; and

(d) Finally, the mean arterial pressure (MAP) is determined by a weighting of the cuff pressures CP-(MAX + 1) and MAPL, as by

$$MAP = (CP(MAX + 1) + (2xMAPL))/2.9 \qquad \text{Eq. 26.}$$

The denominator (2.9 in Eq. 26) may be somewhat lower for operation in a "stat" mode, e.g., 2.85.

The above-discussed algorithm for determining mean arterial pressure is illustrated in Figs. 8 and 9. Step 101 (Fig. 9) finds the peak interval MAX (for example, by execution comparable to Equations 4-10). A processing variable AMP is set equal to the peak value $\phi A(MAX + 1)$ of the complex following the interval MAX (step 105) and the interval MN1 is next determined (step 106) as the first occurring complex less than the value AMP (i.e., $\phi A(MAX + 1)$) to the left of time MAX in Fig. 8 (e.g., by processing comparable to Equations 12-15). An interpolation is then conducted to find the point MAPL (Fig. 8: step 111 in Fig. 9) and the final processing operation 113 finds the subject's mean arterial pressure by implementing Equation 26.

To again illustrate by numerical example from the Fig. 1 data

$$MAPL = 18.7 + ((17.1\text{-}18.7)x(8.3\text{-}5.9))/(8.4\text{-}5.9) = 17.2 \text{ kPa} \qquad \text{Eq. 27.}$$

$$MAP = (13.9 + 2x17.2)/2.9 = 16.7 \text{ kPa} \qquad \text{Eq. 28.}$$

The foregoing discussion has thus demonstrated that measured data may be enhanced by replacing data lost through measurement artifacts or the like or deviations from a proper data pattern by approximated values. Specific data processing algorithms were presented and discussed for the computation of a subject's measured systolic, diastolic and mean arterial blood pressures.

The above-described arrangements are merely illustrative of the principles of the present invention. Numerous modifications and adaptations thereof will be readily apparent to those skilled in the art without departing from the scope of the present invention as claimed. For example, the pressure measurement mode is described above as stepped deflation from an initial inflation above the subject's systolic pressure. The measurement of the instant invention can alternatively be performed by stepped inflation from an initial sub-diastolic cuff pressure; or via continuous vis-a-vis discrete cuff inflation or deflation.

## Claims

1. An automated blood pressure monitor, comprising:

an inflatable cuff;

means for inflating and deflating said cuff;

pressure transducer means coupled to said cuff for cuff pressure (CP(I)) measurement;

means responsive to said cuff pressure measurement (CP(I)) for generating a signal representing blood pressure pulses;

complex peak storing means for storing values ($\phi$A(I)) characterising the peak amplitudes of said detected pulses at different cuff pressures (CP(I));

cuff pressure storing means for storing the cuff pressures (CP(I)) associated with said cuff pressure pulse peak signals ($\phi$A(I)); and

mean arterial pressure (MAP) determining means comprising means (101) for locating the maximum cuff pressure complex peak amplitude ($\phi$A(MAX)) stored in said complex peak storing means; characterised in that:

said mean arterial pressure (MAP) determining means further comprises:

means (105) for locating a successor peak value ($\phi$A(MAX + 1)) stored in said complex peak storing means next occurring after said maximum complex peak amplitude ($\phi$A(MAX));

means (106) for selecting from said complex peak storing means, plural peak amplitudes ($\phi$A(MN1),$\phi$A(MN1 + 1)) generated at higher cuff pressures than that associated with said maximum complex peak amplitude ($\phi$A(MAX)); and

determining means (111,113) for determining mean arterial pressure (MAP) from:

said selected plural peak amplitudes ($\phi$A(MN1),$\phi$A(MN1 + 1) and

cuff pressures (CP(I)) stored in said cuff pressure storing means associated with:

said selected plural peak amplitudes ($\phi$A(MN1),$\phi$A(MN1 + 1)); and

said successor peak value ($\phi$A(MAX + 1)).

2. An automated blood pressure monitor according to claim 1, in which the monitor is digital processor controlled and is operative in conjunction with a stored program.

3. An automated blood pressure monitor according to claim 2, in which the mean arterial pressure determining means further comprises computing means (106) for computing two different cuff pressure levels (MN1,MN1 + 1) each a different fraction of the successor peak value ($\phi$A(MAX + 1)) for selecting said plural peak amplitudes ($\phi$A(MN1),$\phi$A(MN1 + 1)).

4. An automated blood pressure monitor according to any of claims 1 to 3, in which the determining means comprises interpolation means.

5. An automated blood pressure monitor according to any of claims 1 to 4, wherein the determining means comprises means (113) for computing a weighted average of the cuff pressure (CP(I)) stored in said cuff pressure storing means corresponding to said successor complex peak signal ($\phi$A(MAX + 1)) and a measure of the cuff pressures (CP(I)) stored in said cuff pressure storing means corresponding to said selected plural complex peak value ($\phi$A(I)).

6. An automated blood pressure monitor according to any of claims 1 to 5, further including data purifying means (Fig.2) operative upon the complex peak amplitude characterising values stored in said complex peak storing means for oorrecting data inaccuracies.

7. An automated blood pressure monitor according to claim 6, wherein a preselected character ( + 1) is stored in said complex peak storing means to signal an unsuccessful pulse flow peak measurement, and wherein said data correcting means comprises means for examining the contents of said complex peak storing means and responsive to detecting said preselected character for replacing said character with a measure of plural stored complex peak values ($\phi$A(I)) at least one of which was obtained at a cuff pressure (CP(I)) higher than that associated with the preselected character.

8. An automated blood pressure monitor according to claim 6 or claim 7, wherein the replacement

12

character was obtained by the average of the cuff pressures next higher and next lower than that associated with the preselected character.

9. An automated blood pressure monitor according to any of claims 6 to 8, wherein said data purifying means includes means for searching said complex storing means for the occurrence of two equal peak amplitude values arising at successive cuff deflation pressures, and means responsive to said searching means for replacing one of the two stored equal values with a measure of two other values stored in said complex peak storing means.

10. An automated blood pressure monitor according to claim 9, wherein the other values include the next higher or the next lower value stored in said complex peak storing means.

11. An automated blood pressure monitor according to claim 1, wherein:
the monitor is oscillometric;
the cuff pressures (CP(I)) are reduced in predetermined cuff pressure decrement levels;
the complex peaks are oscillation complexes;
the associated cuff pressures are cuff pressure decrement levels;
said monitor further comprises:
means for identifying and maintaining the associated cuff pressure decrement levels;
means for interpolating the peak amplitudes (21i,22i) from the identified associated cuff pressure decrement levels; and
said determining means further comprises:
means (113) for developing mean arterial pressure (MAP) as a predetermined weighted average of the cuff pressure (CP(I)) corresponding to the interpolated peak amplitude and at least one of the identified and maintained cuff pressure decrement levels.

## Revendications

1. Un moyen automatisé de surveillance de pression sanguine comprenant:
un brassard gonflable;
des moyens de gonflage et de degonflage dudit brassard;
des moyens transducteurs de pression couples audit brassard en vue de la mesure de pression de brassard (CP(I));
des moyens sensibles à ladite mesure de pression (CP(I)) pour engendrer un signal représentant des impulsions de pression sanguine;
un moyen de mémorisation de pics de complexes pour mémoriser des valeurs ($\phi$A(I)) caractérisant les amplitudes de pic desdites impulsions détectées à différentes pressions de brassard (CP(I));
des moyens de mémorisation de pression de brassard pour mémoriser les pressions de brassard (CP(I)) associées auxdits signaux ($\phi$A(I)) de pics d'impulsions de pression de brassard; et
un moyen (MAP) de détermination de la pression artérielle moyenne comprenant : des moyens (101) pour situer l'amplitude maximale ($\phi$A(MAX) de pic de complexe de pression de brassard mémorisée dans lesdits moyens de mémorisation de pics de complexes;
caractérisé en ce que:
ledit moyen de détermination de la pression artérielle moyenne (MAP) comprend en outre:
des moyens (105) pour situer une valeur de pic successeur ($\phi$A(MAX + 1)) mémorisée dans lesdits moyens de mémorisation de pics de complexes, se produisant immédiatement après ladite amplitude maximale de pic de complexe ($\phi$A(MAX));
des moyens (106) pour choisir, à partir desdits moyens de mémorisation de pics de complexes, plusieurs amplitudes de pics ($\phi$A(MN1)), ($\phi$A(MN1 + 1)) engendrées à des pressions de brassards supérieures à celle qui est associée à ladite amplitude maximale de pic de complexe ($\phi$A(MAX)); et
un moyen (111, 113) de détermination pour déterminer la pression artérielle moyenne (MAP) à partir:
de ladite pluralité d'amplitudes choisies de pics ($\phi$A(MN1)) ($\phi$A(MN1 + 1)); et
des pressions de brassard (CP(I)) mémorisées dans lesdits moyens de mémorisation de pressions de brassard associées avec:
ladite pluralité de valeurs d'amplitudes choisies de pics ($\phi$A(MN1)) ($\phi$A(MN1 + 1)); et
ladite de valeur de pic successeur ($\phi$A(MAX + 1)).

2. Un moyen automatisé de surveillance de pression sanguine selon la revendication 1, dans lequel le moyen de surveillance est commandé par un processeur numérique et opère en liaison avec un programme mémorisé.

3. Un moyen automatisé de surveillance de pression sanguine selon la revendication 2, dans lequel le moyen de détermination de pression artérielle moyenne comprend en outre des moyens de calcul (106) pour calculer deux niveaux différents de pressions de brassard (MN1, MN + 1) constituant chacun une fraction différente de la valeur de pics successeurs ($\phi$A(MAX + 1)) pour choisir ladite pluralité d'amplitudes de pics (($\phi$A(MN1)) ($\phi$A(MN1 + 1)).

4. Un moyen automatisé de surveillance de pression sanguine selon l'une quelconque des revendications 1 à 3, dans lequel le moyen de détermination comprend un moyen d'interpolation.

5. Un moyen automatisé de surveillance de pression sanguine selon l'une quelconque des revendications 1 à 4, dans lequel le moyen de détermination comprend un moyen (113) pour calculer une moyenne pondérée de la pressions de brassard (CP(I)) mémorisée dans ledit moyen de mémorisation de pressions de brassard correspondant audit signal successeur de pic de complexe ($\phi$A(MAX + 1)) et une mesure des pressions de brassard (CP(I)) mémorisées dans ledit moyen de mémorisation de pressions de brassard correspondant à ladite pluralité de valeur choisies de pics de complexes ($\phi$A(I)).

6. Un moyen automatisé de surveillance de pression sanguine selon l'une quelconque des revendications 1 à 5, comprenant en outre un moyen de purification de données (Fig. 2) agissant sur les valeurs caractérisantes d'amplitudes de pics de complexes mémorisées dans ledit moyen de mémorisation de pics de complexes pour corriger des imprécisions de données.

7. Un moyen automatisé de surveillance de pression sanguine selon la revendication 6, dans lequel un caractère choisi au préalable ( + 1) est mémorisé dans ledit moyen de mémorisation de pics de complexes pour signaler une mesure non réussie de pic de flux d'impulsion, et dans lequel ledit moyen de correction de données comprend un moyen pour examiner le contenu desdits moyens de mémorisation de pics de complexes et sensible à une détection dudit caractère choisi au préalable pour remplacer ledit caractère à l'aide d'une mesure d'une pluralité de valeurs mémorisées de pics de complexes ($\phi$A(I)) dont au moins l'une a été obtenue à une pression de brassard (CP(I)) supérieur à celle qui est associée au caractère choisi au préalable.

8. Un moyen automatisé de surveillance de pression sanguine selon la revendication 6 ou la revendication 7 dans lequel le caractère de remplacement a été obtenu par la moyenne des pressions de brassard immédiatement supérieure et immédiatement inférieure à celle qui est associée au caractère choisi au préalable.

9. Un moyen automatisé de surveillance de pression sanguine selon l'une quelconque des revendications 6 à 8, dans lequel ledit moyen de purification de données comprend un moyen pour explorer ledit moyen de mémorisation de complexe quant a l'apparition de deux valeurs égales d'amplitudes de pics apparaissant à des pressions successives de degonflage de brassard, et des moyens sensibles audit moyen d'exploration pour remplacer l'une des deux valeurs égales mémorisées par une mesure de deux autres valeurs mémorisées dans ledit moyen de mémorisation de pics de complexes.

10. Un moyen automatisé de surveillance de pression sanguine selon la revendication 9, dans lequel les autres valeurs comprennent la valeur immédiatement supérieure ou la valeur immédiatement inférieure mémorisée dans ledit moyen de mémorisation de pics de complexes.

11. Un moyen automatisé de surveillance de pression sanguine selon la revendication 1, dans lequel:
le moyen de surveillance est oscillométrique;
les pressions de brassard (CP(I)) sont réduites selon des niveaux de décrément prédéterminés de diminution de pression de brassard;
les pics de complexes sont des complexes d'oscillation;
les pressions de brassard associées sont des niveaux de décrément de pression de brassard; et
ledit moyen de surveillance comprend en outre:
des moyens pour identifier et maintenir les niveaux associés de décrément de pression de

14

EP 0 208 521 B1

brassard;

des moyens pour interpoler les amplitudes de pics (21i, 22i) à partir de niveaux associés identifiés de décrément de pression de brassard; et

ledit moyen de détermination comprend en outre:

des moyens (113) pour développer une pression artérielle moyenne (MAP) sous forme de moyenne pondérée prédéterminée de la pression de brassard (CP(I)) correspondant à l'amplitude interpolée de pics et au moins a l'un des niveaux identifiés et maintenus de décrément de pression de brassard.

**Patentansprüche**

1. Automatische Blutdrucküberwachungseinrichtung, mit:

einer aufpumpbaren Manschette;

Mitteln zum Aufpumpen und Abpumpen der Manschette;

Druckwandlermitteln, die zur Messung des Manschettendruckes (CP(I)) an die Manschette angeschlossen sind;

Mitteln, die auf die Manschettendruckmessung (CP(I)) ansprechen, um ein die Blutdruckimpulse darstellendes Signal zu erzeugen;

Komplex-Spitzenspeichermitteln zum Speichern von Werten ($\phi$A(I)), welche die Spitzenamplituden der erfaßten Impulse bei verschiedenen Manschettendrücken (CP(I)) kennzeichnen;

Manschettendruckspeichermitteln zum Speichern der Manschettendrücke (CP(I)), die den Manschettendruckimpulsspitzensignalen ($\phi$A(I)) zugeordnet sind; und

Mitteln zur Bestimmung des mittleren arteriellen Druckes (MAP), die Mittel (101) zum Auffinden der maximalen, in den Komplex-Spitzenspeichermitteln gespeicherten Komplex-Manschettendruckspitzenamplitude ($\phi$A(MAX)) aufweisen, dadurch gekennzeichnet, daß:

die Mittel zur Bestimmung des mittleren arteriellen Druckes (MAP) weiters aufweisen:

Mittel (105) zum Auffinden eines Folgespitzenwertes ($\phi$A(MAX + 1)), der in den Komplex-Spitzenspeichermitteln im Anschluß an die maximale Komplex-Spitzenamplitude ($\phi$A(MAX)) gespeichert ist;

Mittel (106) zum Auswählen einer Mehrzahl von Spitzenamplituden ($\phi$A(MN1), $\phi$A(MN1 + 1)) aus den Komplex-Spitzenspeichermitteln, wobei die Spitzenamplituden bei höheren Manschettendrücken erzeugt wurden als jener, welcher der maximalen Komplex-Spitzenamplitude ($\phi$A(MAX)) zugeordnet ist; und

Bestimmungsmittel (111, 113) zur Bestimmung des mittleren arteriellen Druckes (MAP) aus:

der ausgewählten Mehrzahl von Spitzenamplituden ($\phi$(MN1), $\phi$A(MN1 + 1)); und

den in den Manschettendruckspeichermitteln gespeicherten Manschettendrücken (CP(I)), die zugeordnet sind:

der ausgewählten Mehrzahl von Spitzenamplituden ($\phi$A(MN1), $\phi$A(MN1 + 1)); und

dem Folgespitzenwert ($\phi$A(MAX + 1)).

2. Automatische Blutdrucküberwachungseinrichtung nach Anspruch 1, bei welcher die Überwachungseinrichtung digitalprozessorgesteuert und in Verbindung mit einem gespeicherten Programm betreibbar ist.

3. Automatische Blutdrucküberwachungseinrichtung nach Anspruch 2, bei welcher die Mittel zur Bestimmung des mittleren arteriellen Druckes weiters Berechnungsmittel (106) zum Berechnen von zwei verschiedenen Manschettendruckpegeln (MN1, MN1 + 1) aufweisen, wobei jeder ein anderer Bruchteil des Folgespitzenwertes ($\phi$A(MAX + 1)) ist, um die Mehrzahl von Spitzenamplituden ($\phi$A(MN1), $\phi$A-(MN1 + 1)) auszuwählen.

4. Automatische Blutdrucküberwachungseinrichtung nach einem der Ansprüche 1 bis 3, bei welcher die Bestimmungsmittel Interpolationsmittel aufweisen.

5. Automatische Blutdrucküberwachungseinrichtung nach einem der Ansprüche 1 bis 4, bei welcher die Bestimmungsmittel Mittel (113) zum Berechnen eines gewichteten Durchschnittes des in den Manschettendruckspeichermitteln gespeicherten Manschettendruckes (CP(I)) aufweisen, der dem Komplex-Folgespitzensignal ($\phi$A(MAX + 1)) entspricht, und zum Berechnen eines Maßes für die in den Manschettendruckspeichermitteln gespeicherten Manschettendrücke (CP(I)), die der ausgewählten Mehrzahl von Komplex-Spitzenwerten ($\phi$A(I)) entsprechen.

15

6. Automatische Blutdrucküberwachungseinrichtung nach einem der Ansprüche 1 bis 5, welche weiters Datenbereinigungsmittel (Fig. 2) aufweist, die auf die in den Komplex-Spitzenspeichermitteln gespeicherten Komplex-Spitzenamplitudenkennwerte wirken, um Datenungenauigkeiten zu korrigieren.

7. Automatische Blutdrucküberwachungseinrichtung nach Anspruch 6, bei welcher ein vorbestimmtes Zeichen ( + 1) in den Komplex-Spitzenspeichermitteln gespeichert wird, um eine erfolglose Impulsflußspitzenmessung zu signalisieren, und wobei die Datenkorrigiermittel Mittel zum Überprüfen des Inhaltes der Komplex-Spitzenspeichermittel aufweisen, die auf das Erkennen des vorbestimmten Zeichens ansprechen, um dieses Zeichen durch ein Maß der Mehrzahl von gespeicherten Komplex-Spitzenwerte ($\phi$A(I)) zu ersetzen, von denen zumindest einer bei einem Manschettendruck (CP(I)) erhalten wurde, welcher höher ist als jener, der dem vorbestimmten Zeichen zugeordnet ist.

8. Automatische Blutdrucküberwachungseinrichtung nach Anspruch 6 oder 7, bei welcher das Ersetzungszeichen durch den Durchschnitt aus dem nächsthöheren und nächstniedrigeren Manschettendruck des dem vorbestimmten Zeichen zugeordneten Manschettendruckes erhalten wurde.

9. Automatische Blutdrucküberwachungseinrichtung nach einem der Ansprüche 6 bis 8, bei welcher die Datenbereinigungsmittel Mittel zum Durchsuchen der Komplex-Speichermittel auf das Auftreten von zwei gleichen Spitzenamplitudenwerten aufweisen, die bei aufeinanderfolgenden Manschettenabpumpdrücken auftreten, und Mittel aufweisen, die auf die Suchmittel ansprechen, um einen der beiden gespeicherten gleichen Werte durch ein Maß von zwei anderen in den Komplex-Spitzenspeichermitteln gespeicherten Werten zu ersetzen.

10. Automatische Blutdrucküberwachungseinrichtung nach Anspruch 9, bei welcher die anderen Werte den nächsthöheren oder den nächstniedrigeren in den Komplex-Spitzenspeichermitteln gespeicherten Wert beinhalten.

11. Automatische Blutdrucküberwachungseinrichtung nach Anspruch 1, bei welcher:
die Überwachungseinrichtung oszillometrisch ist;
die Manschettendrücke (CP(I)) in vorbestimmten Manschettendruckdekrementpegeln verringert werden;
die Komplex-Spitzen Schwingungskomplexe sind;
die zugeordneten Manschettendrücke Manschettendruckdekrementpegel sind;
die Überwachungseinrichtung weiters aufweist:
Mittel zum Erkennen und Aufrechterhalten der zugeordneten Manschettendruckdekrementpegel;
Mittel zum Interpolieren der Spitzenamplituden (21i, 22i) aus den erkannten zugeordneten Manschettendruckdekrementpegeln; und
wobei die Bestimmungsmittel weiters aufweisen:
Mittel (113) zum Entwickeln des mittleren arteriellen Druckes (MAP) als vorbestimmter gewichteter Durchschnitt des Manschettendruckes (CP(I)), welcher der interpolierten Spitzenamplitude und zumindest einem der erkannten und aufrechterhaltenen Manschettendruckdekrementpegel entspricht.

FIG-1

CP(1)=201 (26.7)
CP(2)=194 (25.9)
CP(6)
CP(8)

ØA(6)
ØA(9)
23.6  22.6  21.9  23.9
21.6  22.9

| CUFF PRESS. CP(I) (TORR) -12 | 201 | 194 | 187 | 168 | 153 | 140 | 128 | 116 | 104 | 93 | 83 | 74 |
|---|---|---|---|---|---|---|---|---|---|---|---|---|
| (kPa) - | 26.7 | 25.9 | 25 | 22.4 | 20.4 | 18.7 | 17.1 | 15.5 | 13.9 | 12.4 | 11.0 | 9.9 |
| MEAS. OSC. AMP. ØA(I)-14 | 0 | 0 | 4 | 1 | 25 | 63 | 63 | 70 | 62 | 53 | 40 | 33 |
| CORRECTED ØA(I)-15 | 0 | 0 | 4 | 14 | 25 | 44 | 63 | 70 | 62 | 53 | 40 | 33 |
| CUFF PRESS. STEP-18 | 1 | 2 | 3 | 4 | 5 | 6 | 7 | 8 | 9 | 10 | 11 | 12 |

ARTIFACT

REPEATED
ØA VALUES

EP 0 208 521 B1

FIG-2
DATA VERIFICATION
ROUTINE

START /10

READ NEXT ØA(I) ~15

ØA(I)=+1? 18

YES

NO

ØA(I)=(ØA(I-1)+ ØA(I+1))/2 23

ØA(I)=ØA(I-1) ? 27

YES

NO

ØA(I-1)=(ØA(I)+ ØA(I-2))/2 30

DONE ? 32

NO

YES

PROCEED

# FIG-3

$23_{MAX}$ $\phi A(MAX)$

$\phi A(L+1)$ @ CP(L+1)

SYSTOLIC

$LVL = \phi A(MAX) * .5$

$\phi A(L)$ @ CP(L)

50 Torr (6.67 kPa)

$22_{MAX}$

$21_{MAX}$

. . . . .

INT. L    INT. L+1    INT. = MAX

# FIG-5

CUFF PRESSURE

SYS.

SYS.

CP(L) - CP(L+1)

CP(L)

CP(L+1)

OSCILLATION AMPLITUDE

$\phi A(L)$    LVL    $\phi A(L+1)$

## FIG-4    SYSTOLIC DE. ?MINATION

```
┌─────────────────────────┐
│   FIND  MAXIMUM         │ ─ 40
│      ØA( I )            │
│  [ØA(MAX.) @ I= MAX.]   │
└─────────────────────────┘
            │
            ▼
┌─────────────────────────┐
│  LVL = ØA(MAX.)✶ 0.5    │ ─ 42
└─────────────────────────┘
            │
            ▼
┌─────────────────────────┐
│  FIND  LARGEST (FIRST)  │ ─ 45
│   ØA(L ) < LVL,L < MAX. │
└─────────────────────────┘
            │
            ▼
┌──────────────────────────────┐
│ SYS. = CP(L)+(CP(L+1)-CP(L))✶ │ ─ 47
│  (LVL-ØA(L))/(ØA(L+1)-ØA(L))  │
└──────────────────────────────┘
```

FIG-6

FIG-8

# FIG-7   DIASTOLIC DETERMINATION

FIND MAXIMUM
$\phi A(I)$
[$\phi A(MAX.), I = MAX.$] — 63

$UDLVL = \phi A(MAX.) * .69$ — 64

$LDLVL = \phi A(MAX.) * 0.55$ — 65

FIND LARGEST (FIRST)
$\phi A(UD) \leq UDLVL$
$UD > MAX.$ — 69

$DAIU = CP(UD) + ((CP(UD-1) - CP(UD)) *$
$(UDLVL - \phi A(UD)) / (\phi A(UD-1) - \phi A(UD))$ — 72

START AT LOWEST CP &
FIND LARGEST (FIRST)
$\phi A(LD) \leq LDLVL$
$LD > MAX.$ — 73

$DIAL = CP(LD) + ((CP(LD-1) - CP(LD)) *$
$(LDLVL - \phi A(LD)) / (\phi A(LD-1) - \phi A(LD))$ — 74

$DIA = (DIAU + DIAL) / 2$ — 82

# FIG-9

FIND MAXIMUM
$\Phi A(I)$
$[\Phi A(MAX.),I=MAX.]$ — 101

AMP = $\Phi A(MAX.+1)$ — 105

FIND LARGEST (FIRST)
$\Phi A(MN1) < AMP$, AND
$MN1 < MAX.$ — 106

$MAPL = CP(MN1) + ((CP(MN1+1)-CP(MN1)) *$
$(AMP-\Phi A(MN1))/(\Phi A(MN1+1)-\Phi A(MN1))$ — 111

$MAP = (CP(MAX.+1)+2*MAPL)/2.9$ — 113